# DEMANDE DE BREVET EUROPEEN

(11) **EP 0 633 003 A1**
(43) Date de publication de la demande: **11.01.1995**
(21) Numéro de dépôt: 94109680.2
(22) Date de dépôt: 23.06.1994
(51) Int. Cl.: A61B 17/39

(54) **Instrument médical d'électrocoagulation**

(30) Priorité: 09.07.1993 CH 2072/93
(71) Demandeur: Parvulesco, Jean, CH-1844 Villeneuve (CH)
(72) Inventeur: Parvulesco, Jean, CH-1844 Villeneuve (CH)
(74) Mandataire: Misrachi, Alfred

(57) **Abrégé**

L'instrument comprend une aiguille (1) en metal comportant un corps tubulaire coudé (2) recouvert d'un revêtement en silicone ou vernis isolant et thermorésistant et une pointe active dénudée (3) constituée par l'extrémité d'un monofil (7) d'alimentation électrique maintenu dans le corps tubulaire et destiné à être relié à un pôle d'une source de courant HF basse tension. Un manche de manutention (5, 6) à l'intérieur duquel passe le monofil (7) est fixé au corps (2) de l'aiguille. Le retour du courant au second pôle de la source est assuré par une plaque conductrice posée sur la peau du patient. L'aiguille (1) est destinée à être introduite dans un vaisseau par une incision mineure de la peau et le tissu lésé d'une varice peut être brûlé par action intraluminale préservant la qualité esthétique de l'intervention.

## Description

La présente invention a pour objet un instrument médical d'électrocoagulation pour le traitement des insuffisances veineuses telles que les varices, comprenant une électrode chauffante pour cautériser par brûlage le tissu corporel.

Le traitement des varices par électrocoagulation se fait habituellement à travers la peau du patient par brûlages ponctuels successifs à l'aide d'instruments utilisés pour l'application des méthodes de thermocoagulation dont l'élément actif est constitué par l'électrode chauffante précitée. Cette méthode thérapeutique permet d'obtenir un effet curatif satisfaisant pour des petites varices mais au prix d'une esthétique médiocre, voire d'une absence totale d'esthétique, par les marques cicatricielles qu'elle laisse sur la peau du patient.

L'invention a pour but de proposer un instrument médical d'électrocoagulation permettant de réaliser une thérapie des insuffisances veineuses telles que les varices qui soit à la fois efficace et esthétique.

A cet effet, l'instrument médical d'électrocoagulation selon l'invention est caractérisé en ce que l'électrode chauffante qu'il comprend est constituée par une aiguille métallique comportant un corps allongé recouvert d'un revêtement en matériau isolant thermorésistant et une pointe active dénudée.

De la sorte, l'électrode ainsi réalisée sous la forme d'une aiguille peut être introduite dans un vaisseau ou une zone corporelle à traiter par une incision cutanée d'étendue minimale de l'ordre du millimètre par exemple dans un pli de flexion de la peau, pour permettre la réalisation d'une endosclérose étagée par introduction totale, sans chauffage de la pointe, suivie d'un retrait progressif avec coagulations par brûlages successifs par la pointe chauffante, jusqu'à éradication complète des zones lésées.

Pendant les temps de coagulation le revêtement du corps de l'aiguille en matériau isolant thermorésistant évite de provoquer des escarres non contrôlées au niveau de l'incision cutanée et préserve ainsi la qualité esthétique de l'intervention.

Pour faciliter la manipulation et le guidage de l'aiguille pendant l'intervention, le corps de l'aiguille peut être avantageusement coudé du côté opposé à la pointe et nanti d'une poignée de manutention.

La longueur, le diamètre et la forme du corps et de la pointe de l'aiguille sont définis en fonction du type d'intervention sur l'idée de base de l'invention de mettre à la disposition du praticien une aiguille transincisionnelle et intraluminale en tant qu'électrode chauffante.

Le dessin annexé représente, à titre d'exemples, deux formes d'exécuion de l'invention.

La figure 1 est une vue d'ensemble partielle de la première forme d'exécution.

Les figures 2, 3 et 4 montrent en détail les pièces constituant l'ensemble représenté figure 1.

La figure 5 représente une pièce de la seconde forme d'exécution.

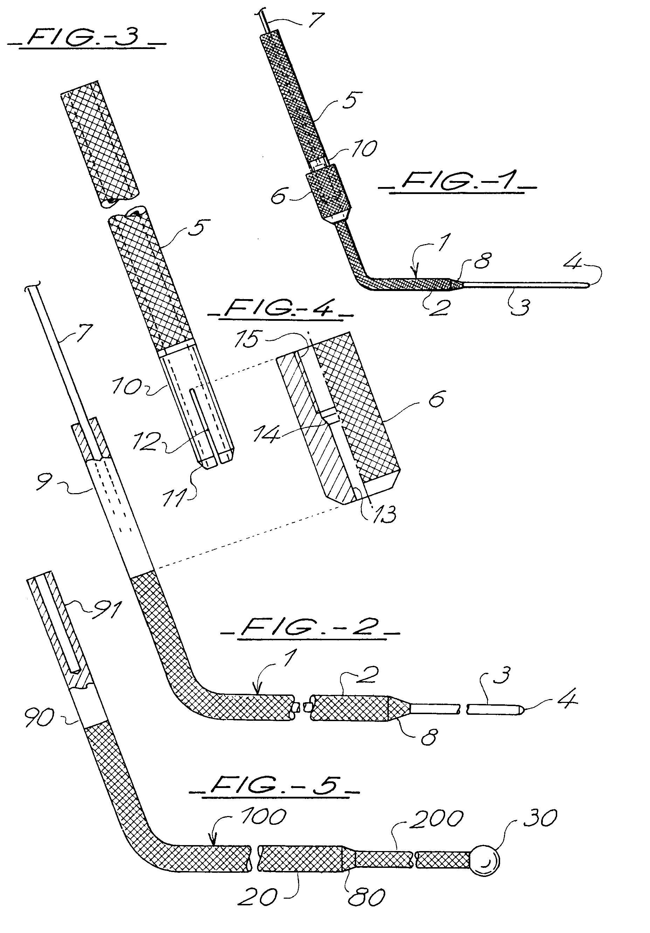

La première forme d'exécution représentée partiellement par les figures 1 à 4 comprend une électrode chauffante constituée par une aiguille 1 composée d'un corps tubulaire coudé 2 et d'une pointe active 3 à bout arrondi 4, et un manche de manutention tubulaire amovible 5 à manchon de fixation 6.

Le corps tubulaire 2 de l'aiguille 1 est métallique, par exemple en acier inoxydable recouvert sur la presque totalité de sa longueur, à partir de la pointe 3 et jusqu'au manchon 6 du manche 5, d'un revêtement en matériau isolant thermorésistant tel que par exemple un silicone ou un vernis isolant supportant des températures de l'ordre de 300° à 500°. Les zones revêtues de ce matériau thermorésistant sont signifiées sur le dessin par des hâchures croisées. Du côté de la pointe 3, l'extrémité du corps 2 de l'aiguille est en forme de tronc de cône 8 dont le petit diamètre est sensiblement égal à celui de son diamètre intérieur, soit au diamètre extérieur de la pointe active 3.

La pointe active 3 est constituée ici par l'extrémité d'un monofil électrique 7 dénudé, par exemple en acier inoxydable également, maintenu à l'intérieur et le long du corps tubulaire 2 de l'aiguille et relié à un pôle d'une source de courant électrique bipolaire basse tension haute fréquence réglable, non représentée, après sa sortie du manche de manutention 5.

Le retour du courant électrique d'alimentation de la pointe active 3 est assuré par une plaque conductrice, non représentée, mise en contact avec le corps du patient, cette plaque conductrice étant reliée au second pôle de la source de courant électrique basse tension haute fréquence.

Le manche de manutention tubulaire 5 est d'un diamètre intérieur permettant l'introduction de l'extrémité dénudée 9 du corps tubulaire 2 de l'aiguille. Du côté de l'aiguille ce manche de manutention tubulaire 5 comporte un filetage extérieur 10 à extrémité tronconique 11 ainsi que quatre fentes axiales 12 équiangulairement espacées et débouchantes.

Le manche de manutention 5 ainsi conçu comporte un manchon 6 de serrage de son extrémité fendue présentant un trou axial 13 de diamètre supérieur à celui du corps 2 de l'aiguille suivi d'une paroi tronconique 14 et d'un filetage 15 intérieurs, ce dernier étant destiné à être vissé sur l'extrémité fendue et filetée 10 du manche 5 pour serrer celle-ci par effet de coin sur l'extrémité dénudée du corps 2 de l'aiguille.

Dans cette première forme d'exécution l'extrémité tronconique 8 du corps 2 de l'aiguille qui précède sa pointe 3 est prévue pour faciliter son introduction dans le vaisseau ou la zone corporelle à traiter par l'incision pratiquée dans la peau du patient.

Comme déjà signalé précédemment, d'autres formes et conceptions d'aiguille peuvent être réalisées selon les besoins du praticien.

La seconde forme d'exécution représentée partiellement par la figure 5 en est un exemple.

Dans cette seconde forme d'exécution qui peut être équipée d'un manche de manutention semblable au manche 5, le corps 20 de l'aiguille 10 est constitué par un fil d'acier inoxydable présentant une extrémité amincie 200 terminée par une pointe active sphérique 30 soudée en bout ou venue de forme, en acier inoxydable également.

Ici également, le passage du diamètre du corps 20 à son extrémité amincie 200 est amorti par une zone en tronc de cône 80.

A l'autre extrémité 90 opposée à la pointe sphérique 30, le corps 20 comporte une partie tubulaire 91 de longueur limitée destinée à la fixation et au contact interne de l'extrémité d'un monofil électrique d'alimentation de la pointe sphérique 30, non représenté.

Le revêtement de l'aiguille 10 en matériau isolant thermorésistant s'étend ici depuis la pointe sphérique 30 jusqu'à l'extrémité 90 destinée à la fixation d'une poignée de manutention.

La pointe sphérique 30 de cette seconde forme d'exécution permet par exemple l'éradication de vaisseaux lésés par une méthode d'intervention mixte de brûlage et d'enlèvement des tronçons brûlés par la protubérance constituée par cette pointe sphérique.

Bien entendu, en variante non représentée, la pointe sphérique 30 peut être soudée en bout d'un monofil d'acier inoxydable passant à l'intérieur et le long d'un corps 20 tubulaire, à la manière de la première forme d'exécution.

De même, dans la première forme d'exécution, la pointe 3 à bout arrondi 4 peut être constituée par un amincissement d'un corps 2 formé par un fil d'acier inoxydable comportant à son autre extrémité une partie tubulaire destinée à la fixation et au contact d'un monofil d'alimentation électrique.

## Revendications

1. Instrument médical d'électrocoagulation pour le traitement des insuffisances veineuses, telles que les varices, comprenant une électrode chauffante pour cautériser par brûlage le tissu corporel, caractérisé en ce que l'électrode chauffante est constituée par une aiguille métallique (1, 100) comprenant un corps allongé (2, 200) recouvert d'un revêtement en matériau isolant thermorésistant et une pointe active dénudée (3, 30).

2. Instrument selon la revendication 1, caractérisé en ce que le corps allongé (2, 200) de l'aiguille est coudé du côté opposé à la pointe active.

3. Instrument selon la revendication 1, caractérisé en ce que le corps allongé de l'aiguille comporte un manche de manutention (5-6).

4. Instrument selon la revendication 3, caractérisé en ce qu'il comporte un manche de manutention amovible (5-6) qui comprend un tube (5) de diamètre intérieur au moins égal à celui du corps de l'aiguille et qui présente à une extrémité un filetage extérieur (10) à fentes axiales (12) et à bout tronconique (11), et un manchon cylindrique (6) de serrage à trou axial (13) de diamètre supérieur à celui du corps de l'aiguille suivi d'une paroi tronconique (14) et d'un filetage (15) intérieurs en prise sur le bout tronconique et le filetage du tube.

5. Instrument selon la revendication 1, caractérisé en ce que la pointe (3) de l'aiguille est allongée et de diamètre uniforme à extrémité arrondie (4).

6. Instrument selon la revendication 1, caractérisé en ce que la pointe (30) de l'aiguille est sphérique.

7. Instrument selon la revendication 1, caractérisé en ce que le corps de l'aiguille comporte au moins une partie tubulaire (9, 90) opposée à la pointe, destinée à la fixation et au contact interne d'un monofil électrique (7) basse tension haute fréquence d'alimentation de la pointe, le retour du courant électrique étant assuré par une plaque conductrice mise en contact avec le corps du patient.

8. Instrument selon les revendications 1, 5 et 7, caractérisé en ce que le corps de l'aiguille est tubulaire et en ce que la pointe à bout arrondi (3, 4) est constituée par l'extrémité dénudée d'un monofil d'alimentation électrique passant par l'intérieur et le long du corps tubulaire de l'aiguille.

9. Instrument selon les revendications 1, 6 et 7, caractérisé en ce que le corps de l'aiguille est tubulaire et en ce que la pointe sphérique (30) est soudée en bout d'un monofil d'alimentation électrique passant par l'intérieur et le long du corps tubulaire de l'aiguille, ledit monofil étant recouvert d'un revêtement en matériau isolant thermorésistant depuis sa sortie du corps tubulaire jusqu'à la pointe sphérique.

10. Instrument selon la revendication 8 ou la revendication 9, caractérisé en ce que l'extrémité du corps tubulaire de l'aiguille jouxtant la pointe est en forme de tronc de cône (8, 80) dont le petit diamètre est sensiblement égal à celui de son diamètre intérieur.
